# EUROPEAN PATENT SPECIFICATION

(11) **EP 3 417 723 B1**
(45) Date of publication and mention of the grant of the patent: **08.04.2026**
(21) Application number: 17753242.1
(22) Date of filing: 15.02.2017
(51) Int. Cl.: A23L 27/20, A23F 5/46, A23L 27/22, A23L 27/00

(54) **COMPOSITION HAVING RICH-TASTE IMPARTING FUNCTION**
ZUSAMMENSETZUNG MIT FUNKTION ZUR VERLEIHUNG EINES VOLLEN GESCHMACKS
COMPOSITION AYANT POUR FONCTION DE CONFÉRER UN GOÛT RICHE

(30) Priority: 16.02.2016 JP 2016027250
(43) Date of publication of application: 26.12.2018
(73) Proprietor: Ajinomoto Co., Inc., Tokyo 104-8315 (JP)
(72) Inventor: YAMAMOTO, Yukiko, Kawasaki-shi Kanagawa 210-8681 (JP); IMADA, Toshifumi, Kawasaki-shi Kanagawa 210-8681 (JP)
(74) Representative: Strehl & Partner mbB
(86) International application number: PCT/JP2017/005580
(87) International publication number: WO 2017/141986

(56) References cited:
- EP-A1- 0 181 421
- WO-A1-2007/055393
- WO-A1-2010/114022
- WO-A1-2011/102477
- WO-A1-2014/083663
- WO-A1-2014/119535
- WO-A1-2015/111462
- WO-A1-2015/199069
- CN-A- 1 911 102
- CN-A- 101 473 865
- JP-A- 2005 269 933
- JP-A- 2007 124 994
- JP-A- 2009 514 791
- JP-A- 2014 039 478
- JP-A- 2015 104 318
- JP-A- 2015 109 858
- JP-A- H04 304 861
- JP-A- S6 344 547
- US-A1- 2010 034 944
- UEDA Y ET AL: "Flavor characteristics of glutathione in raw and cooked foodstuffs", BIOSCI. BIOTECH. BIOCHEM,, vol. 61, no. 12, 1 January 1997 (1997-01-01), pages 1977 - 1980, XP008123781, ISSN: 0916-8451, DOI: 10.1271/BBB.61.1977
- SCHLICHTHERLE-CERNY H ET AL: "IDENTIFICATION OF NONVOLATILE FLAVOR COMPOUNDS BY HYDROPHILIC INTERACTION LIQUID CHROMATOGRAPHY-ELECTROSPRAY MASS SPECTROMETRY", FOOD, SCIENCE AND TECHNOLOGY, MARCEL DEKKER, NEW YORK, NY, US, vol. 131, 1 January 2004 (2004-01-01), pages 363 - 378, XP009050530, ISSN: 0891-8961
- KURODA MOTONAKA ET AL.: "Determination and quantification of the kokumi peptide, gamma- glutamyl-valyl-glycine, in commercial soy sauces", FOOD CHEM., vol. 141, no. 2, 27 March 2013 (2013-03-27), pages 823 - 828, XP028571715

## Description

### Technical Field

The present invention relates to a composition having a "kokumi"-imparting function, and use thereof.

### Background Art

Taste substances have been used in the field of foods for many years. **In** particular, substances having the five basic tastes, i.e., sweet taste, salty taste, sour taste, bitter taste, and umami, and substances that enhance these tastes are widely used as seasonings.

There is known "kokumi" as a concept of taste that cannot be expressed with the aforementioned basic tastes. The term "kokumi" refers to a concept of taste that enhances not only the basic tastes, but also marginal tastes and marginal flavors of the basic tastes, such as thickness, growth (mouthfulness), continuity, and harmony. So far to date, techniques for effectively imparting "kokumi" to foods and drinks have been desired.

As substances that can impart "kokumi" ("kokumi"-imparting substances), there are known, for example, γ-glutamyl tripeptides such as glutathione (γ-Glu-Cys-Gly) and γ-Glu-Val-Gly, and γ-glutamyl dipeptides such as γ-Glu-Met and γ-Glu-Thr (Patent documents 1 and 2). Patent document 2 discloses that these γ-glutamyl peptides can be used also in the form of a pharmaceutically acceptable salt, and examples of the salt include salts with tartaric acid.

There is also known a food or drink containing a γ-glutamyl peptide and fruit juice (Patent document 3). Patent document 3 discloses that this food or drink may further contain an acid, and examples of the acid include tartaric acid and adipic acid.

It is also known that "kokumi" can further be enhanced by a combinatory use of a γ-glutamyl peptide with a dicarboxylic acid selected from succinic acid, maleic acid, and methylmalonic acid (Patent document 4).

However, it is not known that initial taste type "kokumi" is further enhanced by a combinatory use of a γ-glutamyl peptide, such as γ-Glu-Val-Gly, with L-tartaric acid or adipic acid.

### Prior art references

### Patent documents

Patent document 1: Japanese Patent No. 1464928
Patent document 2: International Patent Publication WO2007/055393
Patent document 3: International Patent Publication WO2014/017485
Patent document 4: International Patent Publication WO2014/119535

Furthermore, JP 2015-109858-A discloses salt taste enhancers that contain an amino-carbonyl reactant, and an organic acid, which can be adipic acid, ascorbic acid, citric acid, succinic acid, acetic acid, oxalic acid, tartaric acid, lactic acid and malic acid; the salt taste enhancers are used e.g. in various types of sup and in soy sauce.

### Summary of the Invention

### Object to be Achieved by the Invention

An object of the present invention is to provide a technique for effectively imparting initial taste type "kokumi" to a food or drink.

### Means for Achieving the Object

The inventors of the present invention conducted various researches in order to achieve the aforementioned object. As a result, they found that initial taste type "kokumi" can be further enhanced by a combinatory use of a γ-glutamyl peptide, such as γ-Glu-Val-Gly, with L-tartaric acid or adipic acid, and accomplished the present invention.

That is, the present invention is embodied as follows. A composition containing the following ingredients (A) and (B):
(A) one or more kinds of γ-glutamyl peptides selected from γ-Glu-Val-Gly, γ-Glu-Abu-Gly, and γ-Glu-Abu; and
(B) one or more kinds of dicarboxylic acids selected from L-tartaric acid and adipic acid.

A method for producing a food or drink, the method comprising:
adding the following ingredients (A) and (B) to a food or drink, or a raw material thereof:
(A) one or more kinds of γ-glutamyl peptides selected from γ-Glu-Val-Gly, γ-Glu-Abu-Gly, and γ-Glu-Abu; and
(B) one or more kinds of dicarboxylic acids selected from L-tartaric acid and adipic acid.

A method for imparting initial taste type kokumi to a food or drink, the method comprising:
adding the following ingredients (A) and (B) to the food or drink, or a raw material thereof:
(A) one or more kinds of γ-glutamyl peptides selected from γ-Glu-Val-Gly, y-Glu-Abu-Gly, and γ-Glu-Abu; and
(B) one or more kinds of dicarboxylic acids selected from L-tartaric acid and adipic acid.

### Modes for Carrying out the Invention

Hereafter, the present invention will be explained in detail. Any of the following descriptions concerning the present invention may be used independently, or they may be used in any appropriate combination.

### <1> Composition of the present invention

The composition of the present invention is a composition containing the following ingredients (A) and (B):
(A) one or more kinds of γ-glutamyl peptides selected from γ-Glu-Val-Gly, γ-Glu-Abu-Gly, and γ-Glu-Abu; and
(B) one or more kinds of dicarboxylic acids selected from L-tartaric acid and adipic acid.

In the present invention, the ingredient (A) and the ingredient (B) are also collectively referred to as "active ingredients".

The composition of the present invention has a "kokumi"-imparting function. The term ""kokumi"-imparting function" refers to a function of imparting "kokumi" to an object such as food or drink. Therefore, the composition of the present invention can be used for imparting "kokumi" to a food or drink. That is, one aspect of the composition of the present invention is a "kokumi"-imparting agent. In the present invention, the term "kokumi" refers to a sensation that cannot be expressed with the five basic tastes, i.e., sweet taste, salty taste, sour taste, bitter taste, and umami, and refers to a concept of taste that enhances not only the basic tastes, but also marginal tastes and marginal flavors of the basic tastes, such as thickness, growth (mouthfulness), continuity, and harmony. In the present invention, examples of "impartation of "kokumi"" include enhancement of the basic tastes, and impartation or enhancement of marginal tastes of the basic tastes, such as thickness, growth (mouthfulness), continuity, and harmony, accompanying the enhancement of the basic tastes. Measurement and comparison of "kokumi" can be carried out by, for example, organoleptic evaluation performed by special panelists.

Taste patterns can be classified into, for example, initial taste, middle taste, and aftertaste. For the present invention, initial taste, middle taste, and aftertaste for "kokumi" in the case of a liquid (i.e. in the case of a liquid food or drink) means "kokumi" sensed in the periods of 0 to 1 second, 1 to 3 seconds, and 3 to 5 seconds after eating (i.e. after the food or drink is held in the mouth), respectively. Furthermore, in the present invention, initial taste, middle taste, and aftertaste for "kokumi" in the case of a solid (i.e. in the case of a solid food or drink) means "kokumi" sensed in the periods of 0 to 4 second, 4 to 10 seconds, and 10 to 15 seconds after eating (i.e. after the food or drink is held in the mouth), respectively. In the present invention, the term "solid" refers to a form other than liquid, and also includes paste, gel, and so forth. According to the present invention, initial taste type "kokumi" (i.e. "kokumi" sensed in the period of 0 to 1 second (in the case of a liquid) or 0 to 4 seconds (in the case of a solid) after eating or drinking of a food or drink) of the food or drink can be further enhanced by combinatory use of the ingredient (A) with the ingredient (B) as compared with the case of using the ingredient (A) alone. That is, according to the present invention, an effect of further enhancing initial taste type "kokumi" (i.e. "kokumi" sensed in the period of 0 to 1 second (in the case of a liquid) or 0 to 4 seconds (in the case of a solid) after eating or drinking of a food or drink) of the food or drink can be obtained by a combinatory use of the ingredient (A) with the ingredient (B) as compared with the case of using the ingredient (A) alone. In the present invention, this effect is also referred to as ""kokumi"-boosting effect". Therefore, the composition of the present invention can be used for, for example, imparting initial taste type "kokumi" to a food or drink. That is, one aspect of the composition of the present invention may be, for example, an initial taste type "kokumi"-imparting agent (i.e. a "kokumi"-imparting agent that can impart initial taste type "kokumi" to a food or drink). Although the type of "kokumi" to be enhanced due to the "kokumi"-boosting effect is not particularly limited, it may be, for example, thickness. Middle taste type and/or aftertaste type "kokumi" of a food or drink may be or may not be enhanced by the combinatory use of the ingredient (A) with the ingredient (B) as compared with the case of using the ingredient (A) alone.

### <1-1> Ingredient (A)

The ingredient (A) is a γ-glutamyl peptide selected from γ-Glu-Val-Gly, γ-Glu-Abu-Gly, and γ-Glu-Abu. Outside the scope of the claimed invention, γ-glutamyl peptides are those that impart "kokumi" to a food or drink. Examples of γ-glutamyl peptides include γ-glutamyl tripeptides represented by the general formula: γ-Glu-X-Gly (X represents an amino acid or an amino acid derivative), and γ-glutamyl dipeptides represented by the general formula: γ-Glu-Y (Y represents an amino acid or amino acid derivative). The symbol "y-" used in the aforementioned general formulas means that X or Y binds to glutamic acid via carboxyl group of the γ-position of glutamic acid. As the γ-glutamyl peptide, one kind of γ-glutamyl peptide may be used, or two or more kinds of γ-glutamyl peptides may be used in combination.

Specific examples of the amino acid include, for example, neutral amino acids such as Gly, Ala, Val, Leu, Ile, Ser, Thr, Cys, Met, Asn, Gln, Pro, and Hyp, acidic amino acids such as Asp and Glu, basic amino acids such as Lys, Arg, and His, aromatic amino acids such as Phe, Tyr, and Trp, and other amino acids such as Orn, Sar, Cit, Nva, Nle, Abu, Tau, Hyp, t-Leu, Cle, Aib, Pen, and Hse.

In the present disclosure, the abbreviations of amino acid residues mean the following amino acids.
(1) Gly: glycine
(2) Ala: alanine
(3) Val: valine
(4) Leu: leucine
(5) Ile: isoleucine
(6) Met: methionine
(7) Phe: phenylalanine
(8) Tyr: tyrosine
(9) Trp: tryptophan
(10) His: histidine
(11) Lys: lysine
(12) Arg: arginine
(13) Ser: serine
(14) Thr: threonine
(15) Asp: aspartic acid
(16) Glu: glutamic acid
(17) Asn: asparagine
(18) Gln: glutamine
(19) Cys: cysteine
(20) Pro: proline
(21) Orn: ornithine
(22) Sar: sarcosine
(23) Cit: citrulline
(24) Nva: norvaline
(25) Nle: norleucine
(26) Abu: α-aminobutyric acid
(27) Tau: taurine
(28) Hyp: hydroxyproline
(29) t-Leu: tert-leucine
(30) Cle: cycloleucine
(31) Aib: α-aminoisobutyric acid (2-methylalanine)
(32) Pen: penicillamine
(33) Hse: homoserine

The term "amino acid derivative" refers to any of various derivatives of such amino acids as mentioned above. Examples of the amino acid derivative include, for example, special amino acids, non-natural amino acids, amino alcohols, and amino acids one or more of which functional groups such as terminal carbonyl group, terminal amino group, and thiol group in the case of cysteine are substituted with any one or more of various substituents. Specific examples of the substituents include, for example, an alkyl group, an acyl group, hydroxyl group, amino group, an alkylamino group, nitro group, sulfonyl group, and various protective groups. Specific examples of the amino acid derivative include, for example, Arg(NO₂) (N-γ-nitroarginine), Cys(SNO) (S-nitrocysteine), Cys(S-Me) (S-methylcysteine), Cys(S-allyl) (S-allylcysteine), Val-NH₂ (valinamide), Val-ol (valinol, 2-amino-3-methyl-1-butanol), Met(O) (methionine sulfoxide), and Cys(S-Me)(O) (S-methylcysteine sulfoxide).

Specific examples of the γ-glutamyl peptide include, for example, γ-Glu-Val-Gly, γ-Glu-Abu-Gly, γ-Glu-Nva-Gly, γ-Glu-Abu, and γ-Glu-Nva. The claimed γ-glutamyl peptides are γ-Glu-Val-Gly, γ-Glu-Abu-Gly, and γ-Glu-Abu, and an especially specific example of the γ-glutamyl peptide is γ-Glu-Val-Gly. The structural formula of γ-Glu-Val-Gly (CAS 38837-70-6, also referred to as Gluvalicine) is shown below as the formula (I).

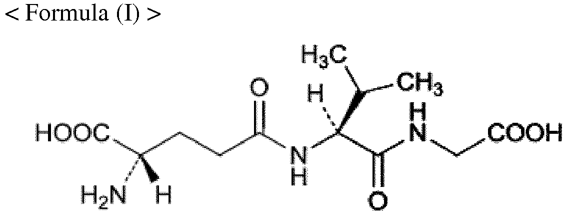

In the present disclosure, the amino acid and amino acid derivative that constitute the γ-glutamyl peptide are L-isomers unless otherwise stated.

In the present disclosure, the γ-glutamyl peptide may be a free compound, a salt, or a mixture of them. That is, the term "y-glutamyl peptide" means a γ-glutamyl peptide as a free compound, a salt thereof, or a mixture of them, unless otherwise stated.

The salt is not particularly limited so long as it can be orally ingested. For example, specific examples of salts for acidic group such as carboxyl group include ammonium salts, salts with alkali metals such as sodium and potassium, salts with alkaline earth metals such as calcium and magnesium, aluminum salts, zinc salts, salts with organic amine such as triethylamine, ethanolamine, morpholine, pyrrolidine, piperidine, piperazine, and dicyclohexylamine, and salts with basic amino acid such as arginine and lysine. Also, for example, specific examples of salts for basic group such as amino group include salts with inorganic acid such as hydrochloric acid, sulfuric acid, phosphoric acid, nitric acid, and hydrobromic acid, salts with organic carboxylic acid such as acetic acid, citric acid, benzoic acid, maleic acid, fumaric acid, tartaric acid, succinic acid, tannic acid, butyric acid, hibenzic acid, pamoic acid, enanthic acid, decanoic acid, teoclic acid, salicylic acid, lactic acid, oxalic acid, mandelic acid, malic acid, methylmalonic acid, and adipic acid, and salts with an organic sulfonic acid such as methanesulfonic acid, benzenesulfonic acid, and p-toluenesulfonic acid. As the salt, one kind of salt may be used, or two or more kinds of salts may be used in combination.

As the γ-glutamyl peptide, commercial products may be used, or those produced in an appropriate manner may be used.

Methods for producing the peptide are not particularly limited, and for example, known methods can be used. Examples of such known methods include (1) a method of chemically synthesizing a peptide, and (2) a method of synthesizing a peptide through an enzymatic reaction. Especially for synthesis of comparatively short peptides of 2 or 3 amino acid residues, it is convenient to use a method of chemically synthesizing a peptide.

When a peptide is chemically synthesized, the peptide can be synthesized or semi-synthesized by using a peptide synthesizer. Examples of the method of chemically synthesizing a peptide include, for example, a solid phase peptide synthesis method. A synthesized peptide can be purified by usual means, for example, ion exchange chromatography, reversed phase high performance liquid chromatography, or affinity chromatography. Such a solid phase peptide synthesis method and the following peptide purification are well known in the art of the present application.

When a peptide is synthesized through an enzymatic reaction, for example, the method described in WO2004/011653 can be used. Specifically, for example, by reacting an amino acid or dipeptide having an esterified or amidated carboxyl group and an amino acid having a free amino group (for example, an amino acid having a protected carboxyl group) in the presence of a peptide synthesis enzyme, a dipeptide or tripeptide can be synthesized. The synthesized dipeptide or tripeptide can be purified as required. Examples of the peptide synthesis enzyme include, for example, a culture broth of a microorganism having an ability to generate a peptide, a culture supernatant separated from such a culture broth, cells separated from such a culture broth, or a processed product of cells of such a microorganism, and peptide synthesis enzymes separated therefrom. As the peptide synthesis enzyme, those appropriately purified can be used as required.

The γ-glutamyl peptide can also be produced by, for example, culturing a microorganism having an ability to produce the γ-glutamyl peptide, and collecting the γ-glutamyl peptide from the culture broth or cells of the microorganism. Specifically, for example, yeast containing a γ-glutamyl peptide such as γ-Glu-Abu at a high concentration can be obtained by the method described in Japanese Patent Laid-open (Kokai) No. 2012-213376. The γ-glutamyl peptide can also be produced by, for example, collecting the γ-glutamyl peptide from an agricultural, fishery, or livestock product containing it.

The γ-glutamyl peptide may be or may not be a purified product. That is, as the γ-glutamyl peptide, a material containing the γ-glutamyl peptide at a high content may be used. The expression "containing a γ-glutamyl peptide at a high content" means that the contained amount of a γ-glutamyl peptide is 100 ppm (w/w) or higher. That is, the expression "blending (adding) a γ-glutamyl peptide" is not limited to cases of blending (adding) the peptide itself, but also includes cases of blending (adding) a material containing the peptide at a high content. Specific examples of such a material containing a γ-glutamyl peptide at a high content include, for example, fermentation products obtained by culturing a microorganism having an ability to produce the peptide such as culture broth, cells, and culture supernatant, agricultural, fishery, or livestock products containing the peptide, and processed products thereof. Examples of the processed products include those obtained by subjecting such fermentation products as mentioned above to a treatment such as concentration, dilution, drying, fractionation, extraction, and purification. Examples of such processed products include, for example, a yeast extract containing a γ-glutamyl peptide such as γ-Glu-Abu (Japanese Patent Laid-open (Kokai) No. 2012-213376). Although there may also be foods and drinks (including food raw materials and seasonings) naturally containing a γ-glutamyl peptide other than yeast extract, such foods and drinks (including food raw materials and seasonings) other than yeast extract themselves may be excluded from the scope of the "material containing a γ-glutamyl peptide at a high content" referred to in the present invention. The γ-glutamyl peptide may be purified to a desired extent. As the γ-glutamyl peptide, for example, a γ-glutamyl peptide having a purity of 50% (w/w) or higher, 70% (w/w) or higher, 90% (w/w) or higher, or 95% (w/w) or higher may be used.

### <1-2> Ingredient (B)

The ingredient (B) is a dicarboxylic acid. The dicarboxylic acid used for the present invention is selected from L-tartaric acid and adipic acid. As the dicarboxylic acid, either one of L-tartaric acid and adipic acid may be used, or both L-tartaric acid and adipic acid may be used.

In the present invention, the dicarboxylic acid may be a free acid, a salt, or a mixture of them. That is, the term "dicarboxylic acid" means a dicarboxylic acid as a free acid, a salt thereof, or a mixture of them, unless otherwise stated. The aforementioned descriptions concerning the salt for acidic groups such as carboxyl group of the γ-glutamyl peptide can be applied *mutatis mutandis* to the salt of the dicarboxylic acid.

As the dicarboxylic acid, commercial products may be used, or those produced in an appropriate manner may be used.

Methods for producing the dicarboxylic acid are not particularly limited, and for example, known methods can be used. For example, the dicarboxylic acid can be produced through chemical synthesis. Specifically, for example, adipic acid can be produced by oxidization of cyclohexanol. The dicarboxylic acid can also be produced by, for example, culturing a microorganism having an ability to produce the dicarboxylic acid, and collecting the dicarboxylic acid from the culture broth or cells of the microorganism. Specifically, for example, adipic acid can be produced by using a microorganism according to the method described in WO2012/137771A1. The dicarboxylic acid can also be produced by, for example, collecting the dicarboxylic acid from an agricultural, fishery, or livestock product containing it. Specifically, for example, L-tartaric acid (free compound) can be collected from tartar obtained upon wine manufacture.

The dicarboxylic acid may be or may not be a purified product. That is, as the dicarboxylic acid, a material containing the dicarboxylic acid at a high content may be used. Although the contained amount of the dicarboxylic acid is not particularly limited so long as the "kokumi"-boosting effect can be obtained, the expression "containing a dicarboxylic acid at a high content" may mean that the contained amount of a dicarboxylic acid is 1% (w/w) or higher, 1.5% (w/w) or higher, 5% (w/w) or higher, or 10% (w/w) or higher. That is, the expression "blending (adding) a dicarboxylic acid" is not limited to cases of blending a dicarboxylic acid itself, but also includes cases of blending a material containing a dicarboxylic acid at a high content. Specific examples of such a material containing a dicarboxylic acid at a high content include, for example, fermentation products obtained by culturing a microorganism having an ability to produce the dicarboxylic acid such as culture broth, cells, and culture supernatant, and processed products thereof. Examples of the processed products include those obtained by subjecting such fermentation products as mentioned above to a treatment such as concentration, dilution, drying, fractionation, extraction, and purification. Specifically, for example, examples of the material containing L-tartaric acid include an isomer mixture such as DL-tartaric acid, and a material containing such a mixture. Although there may be foods and drinks (including food raw materials and seasonings) naturally containing a dicarboxylic acid, such foods and drinks (including food raw materials and seasonings) themselves may be excluded from the scope of the "material containing a dicarboxylic acid at a high content" referred to in the present invention. The dicarboxylic acid may be purified to a desired extent. As the dicarboxylic acid, for example, a dicarboxylic acid having a purity of 50% (w/w) or higher, 70% (w/w) or higher, 90% (w/w) or higher, or 95% (w/w) or higher may be used.

### <1-3> Composition of the present invention

The composition of the present invention contains the aforementioned active ingredients.

When the composition of the present invention contains an ingredient corresponding to both the ingredient (A) and the ingredient (B), such as L-tartaric acid salt or adipic acid salt of a γ-glutamyl peptide selected from γ-Glu-Val-Gly, γ-Glu-Abu-Gly, and γ-Glu-Abu, this ingredient serves as both the ingredient (A) and the ingredient (B) in the composition of the present invention. Namely, when the composition of the present invention contains an ingredient corresponding to both the ingredient (A) and the ingredient (B), such as L-tartaric acid salt or adipic acid salt of a γ-glutamyl peptide selected from γ-Glu-Val-Gly, γ-Glu-Abu-Gly, and γ-Glu-Abu, the composition of the present invention may or may not further contain the ingredient (A) and/or the ingredient (B).

The composition of the present invention may consist only of the aforementioned active ingredients, or may further contain another ingredient. The composition of the present invention may be configured as a seasoning.

The "other ingredient" is not particularly limited so long as it can be orally ingested. As the "other ingredient", for example, those blended and used in seasonings, foods, drinks, or pharmaceuticals can be used.

Examples of the "other ingredient" include, for example, compounds having a "kokumi"-imparting activity and compounds having a calcium receptor-stimulating activity, other than the aforementioned γ-glutamyl peptides. Specific examples of the compounds having a "kokumi"-imparting activity include, for example, alliin. Specific examples of the compounds having a calcium receptor-stimulating activity include, for example, cations such as calcium and gadolinium; basic peptides such as polyarginine and polylysine; polyamines such as putrescine, spermine, and spermidine; proteins such as protamine; peptides such as phenylalanine; and cinacalcet. As also for these compounds, those that can form a salt may be used in the form of a salt. The descriptions concerning the salt of γ-glutamyl peptide mentioned above can be applied *mutatis mutandis* to the salt of these compounds.

Specific examples of the "other ingredient" include, for example, saccharides such as sugar, honey, maple syrup, sucrose, glucose, fructose, isomerized sugars, and oligosaccharides; sugar alcohols such as xylitol and erythritol; highly sweet sweeteners; inorganic salts such as common salt, sodium chloride, and potassium chloride; organic acids such as acetic acid and citric acid, and salts thereof; amino acids such as glutamic acid and glycine, and salts thereof; nucleic acids such as inosinic acid, guanylic acid, and xanthylic acid, and salts thereof; dietary fibers, pH buffers, excipients, fillers, perfumes, edible oils, ethanol, and water. The aforementioned descriptions concerning the salt of γ-glutamyl peptide can be applied *mutatis mutandis* to the salt of these ingredients.

As the "other ingredient", one kind of ingredient may be used, or two or more kinds of ingredients may be used in combination.

The form of the composition of the present invention is not particularly limited. The composition of the present invention may be in any form, such as the form of powder, granule, liquid, paste, or cube.

The concentrations and contained amount ratios of the ingredients (namely, the active ingredients and other optional ingredients) in the composition of the present invention are not particularly limited so long as the "kokumi"-boosting effect can be obtained.

The concentrations and contained amount ratios of the active ingredients in the composition of the present invention can be appropriately determined depending on various conditions such as the types of the active ingredients, the concentrations of the active ingredients at the time of eating or drinking, and the amount of the composition of the present invention to be used.

Although the total concentration of the active ingredients in the composition of the present invention is not particularly limited, for example, it may be 40 ppm (w/w) or higher, 100 ppm (w/w) or higher, 1000 ppm (w/w) or higher, 1% (w/w) or higher, 5% (w/w) or higher, or 10% (w/w) or higher, may be 100% (w/w) or lower, 99.9% (w/w) or lower, 70% (w/w) or lower, 50% (w/w) or lower, 30% (w/w) or lower, 10% (w/w) or lower, 5% (w/w) or lower, or 1% (w/w) or lower, or may be within a range defined as a non-contradictory combination thereof. The term "total concentration of the active ingredients" means the total of the concentration of the ingredient (A) and the concentration of the ingredient (B).

In the composition of the present invention, the ratio (weight ratio) of the contained amount of the ingredient (B) to the contained amount of the ingredient (A) (also defined as: contained amount of the ingredient (B)/contained amount of the ingredient (A)), for example, may be 0.05 or higher, 0.1 or higher, 0.5 or higher, 1 or higher, 1.5 or higher, 3 or higher, 5 or higher, 10 or higher, 20 or higher, 50 or higher, or 100 or higher, may be 5000 or lower, 2000 or lower, 1000 or lower, 500 or lower, or 200 or lower, or may be within a range defined as a combination thereof. Specifically, the ratio of the contained amount of the ingredient (B) to the contained amount of the ingredient (A) (contained amount of the ingredient (B)/contained amount of the ingredient (A)) may be, for example, 0.05 to 5000, or 0.5 to 5000. In the composition of the present invention, it is acceptable that, for example, the ratio (molar ratio) of the contained amount of the ingredient (B) to the contained amount of the ingredient (A) (contained amount of the ingredient (B)/contained amount of the ingredient (A) [mol/mol]) is not 1, i.e., the ratio may be lower than 1 (namely, the contained amount of the ingredient (B) [mol] < the contained amount of the ingredient (A) [mol]), or may be higher than 1 (namely, the contained amount of the ingredient (B) [mol] > the contained amount of the ingredient (A) [mol]). The ratio (molar ratio) of the contained amount of the ingredient (B) to the contained amount of the ingredient (A) (contained amount of the ingredient (B)/contained amount of the ingredient (A) [mol/mol]), for example, may be higher than 0.1, or may be lower than 10000. Specifically, the ratio (molar ratio) of the contained amount of the ingredient (B) to the contained amount of the ingredient (A) (contained amount of the ingredient (B)/contained amount of the ingredient (A) [mol/mol]) may be, for example, higher than 0.1 and lower than 1, or higher than 1 and lower than 10000.

When a material containing the active ingredient is used, the contained amount (concentration) of the active ingredient shall be calculated on the basis of the amount of the active ingredient itself contained in the material. When the active ingredient is in the form of a salt, the contained amount (concentration) of the active ingredient shall be calculated on the basis of the amount of the active ingredient in term of a value obtained by converting the mass of the salt into the mass of the corresponding free compound in an amount equimolar to the salt.

The concentrations of the active ingredients in the composition of the present invention can be determined so as to, for example, satisfy the total concentration and the contained amount ratio of the active ingredients exemplified above.

The contained amount (concentration) of the ingredient (A) in the composition of the present invention may be, for example, such a concentration that the concentration of the ingredient (A), when a food or drink is produced by using the composition of the present invention, comes to be within a desired range at the time of eating or drinking the food or drink. The concentration of the ingredient (A) at the time of eating or drinking may be, for example, within the range mentioned later. The contained amount (concentration) of the ingredient (A) in the composition of the present invention may be, for example, 40 ppm (w/w) or higher, or 65% (w/w) or lower.

The contained amount (concentration) of the ingredient (B) in the composition of the present invention may be, for example, such a concentration that the concentration of the ingredient (B), when a food or drink is produced by using the composition of the present invention, comes to be within a desired range at the time of eating or drinking the food or drink. The concentration of the ingredient (B) at the time of eating or drinking may be, for example, in the range mentioned later.

The ingredients contained in the composition of the present invention (namely, the active ingredients and other optional ingredients) may be contained as a mixture thereof in the composition of the present invention, or may be contained separately as individual ingredients or separately as any combinations thereof in the composition of the present invention. So long as the active ingredients coexist in the food or drink produced by adding the composition of the present invention, the "kokumi"-boosting effect can be obtained.

### <2> Method of the present invention

According to the present invention, "kokumi" can be imparted to a food or drink by using the active ingredients (namely, the ingredient (A) and the ingredient (B)). That is, the method of the present invention is a method for imparting "kokumi" to a food or drink, which comprises adding the active ingredients to a food or drink, or a raw material thereof. In addition, one aspect of the method of the present invention is a method for producing a food or drink, which comprises adding the active ingredients to a food or drink, or a raw material thereof.

When an ingredient corresponding to both the ingredient (A) and the ingredient (B), such as L-tartaric acid salt or adipic acid salt of a γ-glutamyl peptide, is added in the method of the present invention, this ingredient serves as both the ingredient (A) and the ingredient (B) in the method of the present invention. Namely, an ingredient corresponding to both the ingredient (A) and the ingredient (B), such as L-tartaric acid salt or adipic acid salt of a γ-glutamyl peptide selected from γ-Glu-Val-Gly, γ-Glu-Abu-Gly, and γ-Glu-Abu, is added in the method of the present invention, the ingredient (A) and/or the ingredient (B) may or may not be further added.

According to the present invention, "kokumi" can be imparted to a food or drink by, for example, using the composition of the present invention. That is, by adding the composition of the present invention, the active ingredients can be added, and "kokumi" can be thereby imparted to a food or drink. That is, the method of the present invention may be, for example, a method for imparting "kokumi" to a food or drink, which comprises adding the composition of the present invention to the food or drink, or a raw material thereof. In addition, one aspect of the method of the present invention may be, for example, a method for producing a food or drink, which comprises adding the composition of the present invention to the food or drink, or a raw material thereof.

According to the method of the present invention, in particular, initial taste type "kokumi" can be imparted to a food or drink.

A food or drink obtained by the method of the present invention is also referred to as "food or drink of the present invention". Specifically, the food or drink of the present invention is a food or drink to which "kokumi" has been imparted. More specifically, the food or drink of the present invention may be a food or drink to which initial taste type "kokumi" has been imparted. The food or drink of the present invention is, in other words, a food or drink to which the ingredient (A) and the ingredient (B) have been added. "Addition" is also expressed as "blending".

The food or drink is not particularly limited, and it includes any foods and drinks. The food or drink also includes seasonings. The food or drink may be a liquid or solid. Examples of the food or drink include, for example, drinks such as milk, soft drinks, alcoholic beverages, and soups; processed meat products such as hams, sausages, Chinese meat dumplings, Chinese steamed meat dumplings, hamburg steaks, deep-fried foods, and pork cutlets; processed aquatic products such as kamaboko (boiled fish paste) and chikuwa (fishcake tube); dairy products such as butter, fermented milk, dried milk, white sauce, yogurt, and custard; rice processed foods such as fried rice; seasonings such as natural type seasonings, flavor seasonings, seasonings for menus, mayonnaise, dressings, and sauces; confectioneries such as cakes and mousses; other processed foods such as breads, noodles, gratins, and croquettes; frozen foods, and so forth. The term "soft drink" refers to a non-alcoholic drink (i.e. a drink of which the alcohol concentration is lower than 1%) other than milk and dairy products. Specific examples of soft drinks include, for example, water, fruits juices, vegetable juices, tea drinks, coffee drinks (such as coffee, and milk beverages containing coffee), carbonated drinks (such as carbonated lemon drinks), and isotonic drinks (sports drinks). Specific examples of soups include, for example, consomme soups (such as those of chicken, pork, and beef), soups containing egg, soups containing wakame seaweed, soups containing dried shark fin, Chinese style soups, curry flavor soups, Japanese clear soups, miso soups, and potage soups. The term "natural type seasoning" refers to a seasoning produced by using a natural product as a raw material through such a process as extraction, decomposition, heating, and fermentation. The term "flavor seasoning" refers to a seasoning to be used for imparting aroma, flavor, and/or taste of a flavor raw material to a food or drink, and it is produced by, for example, adding sugar, common salt, or the like to a natural type seasoning. Specific examples of flavor seasonings include, for example, seasonings having bonito flavor, chicken flavor, pork flavor, beef flavor, or the like. The term "seasoning for menus" refers to a seasoning suitable for cooking of a specific menu (such as Chinese menus). Examples of frozen foods include frozen products of such foods and drinks as exemplified above. Specific examples of frozen foods include, for example, Chinese meat dumplings, Chinese steamed meat dumplings, fried rice, hamburg steaks, deep-fried foods, gratins, pork cutlets, croquettes, cakes, and mousses. Modes for providing the food or drink are not particularly limited. The food or drink may be or may not be provided in a state that the food or drink can be eaten or drunk as it is. The food or drink may be, for example, prepared into a state suitable for eating or drinking before or at the time of eating or drinking, and then eaten or drunk. For example, in the case of a drink such as coffee drink, it may be provided as a drink contained in a container that can be drunk as it is (such as canned coffee), or may be provided as a concentrate such as powder that is diluted and then drunk (such as stick coffee). The food or drink is not limited to usual foods and drinks, and it also includes so-called health foods and foods for medical use, such as supplements, foods with nutrient function claims, and foods for specified health uses. For example, such foods and drinks as exemplified above may each be provided as a usual food or drink, or may each be provided as a health food or a food for medical use. Incidentally, a method wherein the food or drink is a food or drink containing fruit-juice may be excluded from the scope of the method of the present invention.

The food or drink of the present invention can be produced by the same methods using the same raw materials as those used for production of usual foods and drinks except that the composition of the present invention or the active ingredients is/are added. The composition of the present invention or the active ingredients may be added at any stage of the manufacturing process of the food or drink. That is, the composition of the present invention or the active ingredients may be added to a raw material of the food or drink, may be added to the food or drink in the middle of the manufacture thereof, or may be added to the completed food or drink. The composition of the present invention or the active ingredients may be added once, or may be added two or more times as divided portions. When the composition of the present invention is added, and the composition of the present invention contains the active ingredients separately as individual ingredients or separately as any combinations thereof, the active ingredients may be simultaneously added to the food or drink, or a raw material thereof, or may be added separately as individual ingredients or separately as any combinations thereof to the food or drink, or a raw material thereof. When the active ingredients are added, the active ingredients may be added simultaneously to the food or drink, or a raw material thereof, or may be added separately as individual ingredients or separately as any combinations thereof to the food or drink, or a raw material thereof.

The method of the present invention may further comprise adding another ingredient (an ingredient other than the active ingredients). The descriptions concerning the "other ingredient" mentioned above for the composition of the present invention can be applied *mutatis mutandis* to the "other ingredient" referred to here. Furthermore, the composition of the present invention may also be used in combination with the "other ingredient". When the "other ingredient" is added, the "other ingredient" may be added in the same manner as that for the composition of the present invention or the active ingredients. For example, the "other ingredient" and the composition of the present invention or the active ingredients may be simultaneously added to a food or drink, or a raw material thereof, or may be added separately as individual ingredients or separately as any combinations thereof to a food or drink, or a raw material thereof.

The addition amounts and addition ratios of the ingredients (namely, the active ingredients and other optional ingredients) in the method of the present invention are not particularly limited so long as the "kokumi"-boosting effect can be obtained.

When the active ingredients are added, the addition amounts and addition ratios of the active ingredients can be appropriately determined according to various conditions such as the types of the active ingredients, and the ingestion manner of the food or drink of the present invention.

The ingredient (A) may be added to a food or drink or a raw material thereof, for example, so that the concentration of the ingredient (A) at the time of eating or drinking is within a desired range. The concentration of the ingredient (A) at the time of eating or drinking, for example, may be 0.0005 ppm (w/w) or higher, 0.005 ppm (w/w) or higher, 0.01 ppm (w/w) or higher, 0.1 ppm (w/w) or higher, 1 ppm (w/w) or higher, or 3 ppm (w/w) or higher, may be 200 ppm (w/w) or lower, 100 ppm (w/w) or lower, 50 ppm (w/w) or lower, or 20 ppm (w/w) or lower, or may be within a range defined as a combination thereof. The concentration of the ingredient (A) at the time of eating or drinking may be specifically, for example, 0.0005 to 200 ppm (w/w), preferably 0.005 to 100 ppm (w/w), more preferably 0.01 to 50 ppm (w/w), further preferably 0.1 to 20 ppm (w/w).

The ingredient (B) may be added to a food or drink or a raw material thereof, for example, so that the concentration of the ingredient (B) at the time of eating or drinking is within a desired range. The concentration of the ingredient (B) at the time of eating or drinking, for example, may be 0.1 ppm (w/w) or higher, 0.5 ppm (w/w) or higher, 1 ppm (w/w) or higher, 3 ppm (w/w) or higher, 5 ppm (w/w) or higher, 10 ppm (w/w) or higher, or 20 ppm (w/w) or higher, may be 5000 ppm (w/w) or lower, 2000 ppm (w/w) or lower, 1000 ppm (w/w) or lower, 500 ppm (w/w) or lower, 200 ppm (w/w) or lower, or 100 ppm (w/w) or lower, or may be within a range defined as a combination thereof. The concentration of the ingredient (B) at the time of eating or drinking may be specifically, for example, 0.1 to 5000 ppm (w/w), preferably 1 to 1000 ppm (w/w), more preferably 5 to 100 ppm (w/w). The concentration of the ingredient (B) at the time of eating or drinking may be or may not be within such a concentration range that addition of the ingredient (B) alone at a concentration within the range does not affect taste or flavor.

The concentrations of the active ingredients at the time of eating or drinking exemplified above may be used as the addition amounts of the corresponding active ingredients as they are, or with appropriate modifications depending on eating or drinking manner of the food or drink. That is, when a food or drink to be eaten or drunk without concentration or dilution (for example, a food or drink to be eaten or drunk as it is) is produced, the concentrations of the active ingredients at the time of eating or drinking exemplified above may be read as they are as the addition amounts of the corresponding active ingredients. Alternatively, when a food or drink to be eaten or drunk after concentration or dilution is produced, the addition amounts of the active ingredients can be determined from the concentrations of the corresponding active ingredients at the time of eating or drinking exemplified above and the magnitude of the concentration or dilution. For example, when a food or drink that is eaten or drunk after dilution of 10 times is produced, 10 times of the concentrations of the active ingredients at the time of eating or drinking exemplified above may be used as the addition amounts of the corresponding active ingredients.

In the method of the present invention, the ratio (weight ratio) of the addition amount of the ingredient (B) to the addition amount of the ingredient (A) (also defined as: addition amount of the ingredient (B)/addition amount of the ingredient (A)), for example, may be 0.05 or higher, 0.1 or higher, 0.5 or higher, 1 or higher, 1.5 or higher, 3 or higher, 5 or higher, 10 or higher, 20 or higher, 50 or higher, or 100 or higher, may be 5000 or lower, 2000 or lower, 1000 or lower, 500 or lower, or 200 or lower, or may be within a range defined as a combination thereof. Specifically, the ratio of the addition amount of the ingredient (B) to the addition amount of the ingredient (A) (addition amount of the ingredient (B)/addition amount of the ingredient (A)) may be, for example, 0.05 to 5000, or 0.5 to 5000. In the composition of the present invention, it is acceptable that, for example, the ratio (molar ratio) of the addition amount of the ingredient (B) to the addition amount of the ingredient (A) (addition amount of the ingredient (B)/addition amount of the ingredient (A) [mol/mol]) is not 1, i.e., the ratio may be lower than 1 (namely, the addition amount of the ingredient (B) [mol] < the addition amount of the ingredient (A) [mol]), or may be higher than 1 (namely, the addition amount of the ingredient (B) [mol] > the addition amount of the ingredient (A) [mol]). The ratio (molar ratio) of the addition amount of the ingredient (B) to the addition amount of the ingredient (A) (addition amount of the ingredient (B)/addition amount of the ingredient (A) [mol/mol]), for example, may be higher than 0.1, or may be lower than 10000. Specifically, the ratio (molar ratio) of the addition amount of the ingredient (B) to the addition amount of the ingredient (A) (addition amount of the ingredient (B)/addition amount of the ingredient (A) [mol/mol]) may be, for example, higher than 0.1 and lower than 1, or higher than 1 and lower than 10000.

When a material containing the active ingredient is used, the addition amount (concentration) of the active ingredient shall be calculated on the basis of the amount of the active ingredient itself contained in the material. When the active ingredient is in the form of a salt, the addition amount (concentration) of the active ingredient shall be calculated on the basis of the amount of the active ingredient in term of a value obtained by converting the mass of the salt into the mass of the corresponding free compound in an amount equimolar to the salt.

When the composition of the present invention is added, the addition amount thereof is not particularly limited so long as the "kokumi"-boosting effect can be obtained. The addition amount of the composition of the present invention can be appropriately determined depending on various conditions such as the types of the active ingredients, the concentrations of the active ingredients in the composition of the present invention, and the ingestion manner of the food or drink. For example, the composition of the present invention may be added to a food or drink or a raw material thereof at an amount of 1 ppm (w/w) to 50% (w/w), or 10 ppm (w/w) to 10% (w/w). The composition of the present invention may also be added to a food or drink or a raw material thereof, for example, so that the concentrations of the active ingredients at the time of eating or drinking come to be in the ranges of the concentrations of the corresponding active ingredients at the time of eating or drinking exemplified above.

### Examples

The present invention will be further specifically explained with reference to the following examples. However, it should not be construed in any sense that these examples are mentioned with the intension of limiting the scope of the present invention. The unit "ppm" used in the examples means "ppm (w/w)".

The γ-glutamyl peptides used for the experiments were obtained as follows. That is, γ-Glu-Val-Gly was synthesized according to the method described in WO2015/133547. γ-Glu-Abu-Gly was obtained from Bachem Feinchemikalien. γ-Glu-Abu was obtained from Sigma-Aldrich.

### Example 1: Effect of combined use of γ-Glu-Val-Gly and L-tartaric acid or adipic acid in milk coffee

As an evaluation system, milk coffee ("Birdy Robusta", Ajinomoto Co., (Thailand) Ltd.) was used. To this milk coffee, 0.2 ppm of γ-Glu-Val-Gly was added, to prepare a control sample. To the control sample, each of the ingredients to be evaluated (Table 1) was further added, to prepare evaluation samples. All of the ingredients to be evaluated were free compounds unless they are described as salt. The concentrations of the ingredients to be evaluated were concentrations lower than the thresholds of concentrations of the respective ingredients that affect taste or flavor of the milk coffee when they are independently added to the milk coffee (i.e. concentrations of the respective ingredients that affect neither the taste nor flavor, when they are independently added to the milk coffee).

Organoleptic evaluation was performed by 2 to 4 persons of special panelists for strength of "coffee-like thickness" of each sample, to determine thickness-enhancing effects (thickness-boosting effect) of the ingredients to be evaluated. The organoleptic evaluation was performed by a four grade scoring method for initial taste, middle taste, and aftertaste (sensed in the periods of 0 to 1 second, 1 to 3 seconds, and 3 to 5 seconds after drinking, respectively, which periods are defined for a liquid) with scores of 0 to 3, wherein score 0 means no effect (i.e. strength of the thickness is the same as that of the control sample), score 1 means weakly effective, score 2 means effective, and score 3 means strongly effective.

The results are shown in Table 1. Thickness-enhancing effect for the initial taste was observed for L-tartaric acid and adipic acid.

**Table 1: Effect of combined use of γ-Glu-Val-Gly and L-tartaric acid or adipic acid in milk coffee**

| Evaluated ingredient | Coffee-like thickness (initial taste) | Coffee-like thickness (middle to aftertaste) |
|---|---|---|
| Succinic acid, 5 ppm | 0 | 2.5 |
| Proline, 12.5 ppm | 0 | 1.5 |
| Potassium dihydrogenphosphate, 3 ppm | 0 | 0.5 |
| Dipotassium hydrogenphosphate, 12.5 ppm | 0 | 1.0 |
| L-Tartaric acid, 6 ppm | 1.5 | 0 |
| Adipic acid, 25 ppm | 1.0 | 0 |
| Citric acid, 3.5 ppm | 0 | 0 |
| Calcium chloride, 50 ppm | 0 | 0 |
| Magnesium chloride, 12.5 ppm | 0 | 0 |
| DL-Malic acid, 3 ppm | 0 | 0 |
| Trisodium citrate, 0.8 ppm | 0 | 0 |
| Sodium fumarate, 1.5 ppm | 0 | 0 |

### Example 2: Effect of combined use of γ-Glu-Val-Gly and L-tartaric acid or adipic acid in carbonated lemon drink and potage soup

As evaluation systems, a carbonated lemon drink (trial product, sugarless, highly sweet sweetener was used) and a potage soup ("Potage", Knorr) were used. In the case of the carbonated lemon drink, 15 ppm of γ-Glu-Val-Gly was added thereto, to prepare a control sample. **In** the case of the potage soup, 2 ppm of γ-Glu-Val-Gly was added, to prepare a control sample. To the control samples, each of the ingredients to be evaluated (Table 2) was further added thereto, to prepare evaluation samples. All of the ingredients to be evaluated were free compounds. The concentrations of the ingredients to be evaluated were concentrations lower than the thresholds of concentrations of the respective ingredients that affect taste or flavor of the evaluation systems when they are independently added to the evaluation systems (i.e. concentrations of the respective ingredients that affect neither the taste nor flavor of the evaluation systems, when they are independently added to the evaluation systems).

Organoleptic evaluation was performed for strength of "thickness" of each sample by 2 persons of special panelists with the same criteria as those of Example 1 wherein score 0 was defined to mean the strength of thickness of each control sample, to determine thickness-enhancing effects (thickness-boosting effects) of the ingredients to be evaluated.

The results are shown in Table 2. For both the evaluation systems, thickness-enhancing effect for initial taste was observed with L-tartaric acid and adipic acid whereas thickness-enhancing effect was not observed with citric acid, as with the case of using the milk coffee as the evaluation system.

**Table 2: Effect of combined use of γ-Glu-Val-Gly and L-tartaric acid or adipic acid in carbonated lemon drink and potage soup**

| <Carbonated lemon drink> | | |
|---|---|---|
| Evaluated ingredient | Thickness (initial taste) | Thickness (middle to aftertaste) |
| L-Tartaric acid, 24 ppm | 1.0 | 0 |
| Adipic acid, 50 ppm | 1.5 | 0 |
| Citric acid, 3.5 ppm | 0 | 0 |

### <Potage soup>

| Evaluated ingredient | Salty taste-like thickness (initial taste) | Salty taste-like thickness (middle to aftertaste) |
|---|---|---|
| L-Tartaric acid, 6 ppm | 1.3 | 0 |
| Adipic acid, 25 ppm | 1.3 | 0 |
| Citric acid, 2 ppm | 0 | 0 |

### Example 3: Effect of combined use of other γ-glutamyl peptides and L-tartaric acid or adipic acid in milk coffee

As an evaluation system, milk coffee ("Birdy Robusta", Ajinomoto Co., (Thailand) Ltd.) was used. To this milk coffee, 0.8 ppm pf γ-Glu-Abu or 0.6 ppm of y-Glu-Abu-Gly was added, and each of the ingredients to be evaluated (Table 3) was further added, to prepare evaluation samples. All of the ingredients to be evaluated were free compounds.

Organoleptic evaluation was performed for strength of "coffee-like thickness" of each sample by 2 persons of special panelists with the same criteria as those of Example 1 wherein score 0 was defined to mean the strength of thickness of each control sample, to determine thickness-enhancing effects (thickness-boosting effects) of the ingredients to be evaluated.

The results are shown in Table 3. For both the γ-glutamyl peptides, thickness-enhancing effect for initial taste by L-tartaric acid or adipic acid was observed, as with the case of using γ-Glu-Val-Gly.

**Table 3: Effect of combined use of γ-glutamyl peptide and L-tartaric acid or adipic acid in milk coffee**

| γ-Glutamyl peptide | Evaluated ingredient | Coffee-like thickness (initial taste) | Coffee-like thickness (middle to aftertaste) |
|---|---|---|---|
| γ-Glu-Abu 0.8 ppm | L-Tartaric acid, 6 ppm | 1.5 | 0 |
| | Adipic acid, 25 ppm | 1.0 | 0.5 |
| | Citric acid, 3.5 ppm | 0 | 0 |
| γ-Glu-Abu-Gly 0.6 ppm | L-Tartaric acid, 6 ppm | 1.5 | 0 |
| | Adipic acid, 25 ppm | 1.0 | 1.0 |
| | Citric acid, 3.5 ppm | 0 | 0 |

### Example 4: Effect of combined use of γ-Glu-Val-Gly and L-tartaric acid or adipic acid in cookie

As an evaluation system, a cookie (trial product, as oil and fat, 90% of shortening and 10% of salt free butter were used) was used. γ-Glu-Val-Gly (5 ppm) was added to dough, to prepare a cookie as a control sample. γ-Glu-Val-Gly (5 ppm) and each of the ingredients to be evaluated (Table 4) were added to dough, to prepare cookies as evaluation samples. All of the ingredients to be evaluated were free compounds unless they are described as salt. The concentrations of the ingredients to be evaluated were concentrations lower than the thresholds of concentrations of the respective ingredients that affect taste or flavor of the evaluation system when they are independently added to the evaluation system (i.e. concentrations of the respective ingredients that affect neither the taste nor flavor, when they are independently added to the evaluation system).

Organoleptic evaluation was performed by 2 persons of special panelists for strength of "thickness" of each sample, to determine thickness-enhancing effects (thickness-boosting effect) of the ingredients to be evaluated. The organoleptic evaluation was performed by a four grade scoring method for initial taste, middle taste, and aftertaste (sensed in the periods of 0 to 4 second, 4 to 10 seconds, and 10 to 15 seconds after eating, respectively, which periods are defied for a solid) with scores of 0 to 3, wherein score 0 means no effect (i.e. strength of the thickness is the same as that of the control sample, score 1 means weakly effective, score 2 means effective, and score 3 means strongly effective.

The results are shown in Table 4. Thickness-enhancing effect for the initial taste was observed for L-tartaric acid and adipic acid.

**Table 4: Effect of combined use of γ-Glu-Val-Gly and L-tartaric acid or adipic acid in cookie**

| Evaluated ingredient | Thickness (initial taste) | Thickness (middle to aftertaste) |
|---|---|---|
| L-Tartaric acid, 5 ppm | 1.5 | 1.0 |
| Sodium L-tartrate, 2 ppm | 0.7 | 0.5 |
| Adipic acid, 20 ppm | 1.2 | 1.0 |
| Citric acid, 1 ppm | 0 | 0 |

### Industrial Applicability

According to the present invention, a composition that can effectively impart initial taste-type "kokumi" to a food or drink can be provided. In addition, according to the present invention, a food or drink to which initial taste-type "kokumi" has been imparted can be produced.

## Claims

1. A composition containing the following ingredients (A) and (B):
(A) one or more kinds of γ-glutamyl peptides selected from γ-Glu-Val-Gly, γ-Glu-Abu-Gly, and γ-Glu-Abu;
(B) one or more kinds of dicarboxylic acids selected from L-tartaric acid and adipic acid.

2. The composition according to claim 1, wherein the ratio of the contained amount of the ingredient (B) to the contained amount of the ingredient (A) is 0.05 to 5000 in terms of weight ratio.

3. The composition according to claim 1 or 2, wherein the contained amount of the ingredient (A) is 40 ppm (w/w) or higher.

4. The composition according to any one of claims 1 to 3, wherein the ingredient (A) consists of γ-Glu-Val-Gly.

5. The composition according to any one of claims 1 to 4, which is a kokumi-imparting agent.

6. The composition according to any one of claims 1 to 5, which is an initial taste type kokumi-imparting agent.

7. A method for producing a food or drink, the method comprising:
adding the following ingredients (A) and (B) to a food or drink, or a raw material thereof:
(A) one or more kinds of γ-glutamyl peptides selected from γ-Glu-Val-Gly, γ-Glu-Abu-Gly, and γ-Glu-Abu;
(B) one or more kinds of dicarboxylic acids selected from L-tartaric acid and adipic acid.

8. The method according to claim 7, wherein the ratio of the addition amount of the ingredient (B) to the addition amount of the ingredient (A) is 0.05 to 5000 in terms of weight ratio.

9. The method according to claim 7 or 8, wherein the ingredient (A) consists of γ-Glu-Val-Gly.

10. The method according to any one of claims 7 to 9, wherein the ingredient (A) is added so that the concentration of the ingredient (A) at the time of eating or drinking is 0.0005 to 200 ppm (w/w).

11. The method according to any one of claims 7 to 10, wherein the ingredient (B) is added so that the concentration of the ingredient (B) at the time of eating or drinking is 0.1 to 5000 ppm (w/w).

12. The method according to any one of claims 7 to 11, wherein the food or drink to be produced is a food or drink imparted with kokumi.

13. The method according to any one of claims 7 to 12, wherein the food or drink to be produced is a food or drink imparted with initial taste type kokumi.

14. A method for imparting initial taste type kokumi to a food or drink, the method comprising:
adding the following ingredients (A) and (B) to the food or drink, or a raw material thereof:
(A) one or more kinds of γ-glutamyl peptides selected from γ-Glu-Val-Gly, γ-Glu-Abu-Gly, and γ-Glu-Abu;
(B) one or more kinds of dicarboxylic acids selected from L-tartaric acid and adipic acid.

## Patentansprüche

1. Zusammensetzung, enthaltend die folgenden Inhaltsstoffe (A) und (B):
(A) eine oder mehrere Arten von unter γ-Glu-Val-Gly, γ-Glu-Abu-Gly und γ-Glu-Abu ausgewählten γ-Glutamylpeptiden;
(B) eine oder mehrere Arten von unter L-Weinsäure und Adipinsäure ausgewählten Dicarbonsäuren.

2. Zusammensetzung nach Anspruch 1, wobei das Gewichtsverhältnis des Gehalts des Inhaltsstoffs (B) zum Gehalt des Inhaltsstoffs (A) 0,05 bis 5000 beträgt.

3. Zusammensetzung nach Anspruch 1 oder 2, wobei der Gehalt des Inhaltsstoffs (A) 40 ppm (Gew./Gew.) oder höher ist.

4. Zusammensetzung nach einem der Ansprüche 1 bis 3, wobei der Inhaltsstoff (A) aus γ-Glu-Val-Gly besteht.

5. Zusammensetzung nach einem der Ansprüche 1 bis 4, welche ein Kokumi-verleihendes Mittel ist.

6. Zusammensetzung nach einem der Ansprüche 1 bis 5, welche ein Kokumi-verleihendes Mittel vom Angeschmack-Typ ist.

7. Verfahren zur Herstellung eines Nahrungsmittels oder Getränks, umfassend: Zugabe der folgenden Inhaltsstoffe (A) und (B) zu einem Nahrungsmittel oder Getränk oder einem Rohmaterial davon:
(A) eine oder mehrere Arten von unter γ-Glu-Val-Gly, γ-Glu-Abu-Gly und γ-Glu-Abu ausgewählten γ-Glutamylpeptiden;
(B) eine oder mehrere Arten von unter L-Weinsäure und Adipinsäure ausgewählten Dicarbonsäuren.

8. Verfahren nach Anspruch 7, wobei das Gewichtsverhältnis der Zugabemenge des Inhaltsstoffs (B) zur Zugabemenge des Inhaltsstoffs (A) 0,05 bis 5000 beträgt.

9. Verfahren nach Anspruch 7 oder 8, wobei der Inhaltsstoff (A) aus γ-Glu-Val-Gly besteht.

10. Verfahren nach einem der Ansprüche 7 bis 9, wobei der Inhaltsstoff (A) so zugegeben wird, dass die Konzentration des Inhaltsstoffs (A) zum Zeitpunkt des Essens oder Trinkens 0,0005 bis 200 ppm (Gew./Gew.) beträgt.

11. Verfahren nach einem der Ansprüche 7 bis 10, wobei der Inhaltsstoff (B) so zugegeben wird, dass die Konzentration des Inhaltsstoffs (B) zum Zeitpunkt des Essens oder Trinkens 0,1 bis 5000 ppm (Gew./Gew.) beträgt.

12. Verfahren nach einem der Ansprüche 7 bis 11, wobei das zu erzeugende Nahrungsmittel oder Getränk ein Kokumi-verleihendes Nahrungsmittel oder Getränk ist.

13. Verfahren nach einem der Ansprüche 7 bis 12, wobei das zu erzeugende Nahrungsmittel oder Getränk ein Kokumi-verleihendes Nahrungsmittel oder Getränk vom Angeschmack-Typ ist.

14. Verfahren zur Verleihung von Angeschmack-Typ Kokumi an ein Nahrungsmittel oder Getränk, umfassend:
Zugabe der folgenden Inhaltsstoffe (A) und (B) zu dem Nahrungsmittel oder Getränk oder einem Rohmaterial davon:
(A) eine oder mehrere Arten von unter γ-Glu-Val-Gly, γ-Glu-Abu-Gly und γ-Glu-Abu ausgewählten γ-Glutamylpeptiden;
(B) eine oder mehrere Arten von unter L-Weinsäure und Adipinsäure ausgewählten Dicarbonsäuren.

## Revendications

1. Composition contenant les ingrédients suivants (A) et (B) :
(A) un ou plusieurs types de peptides γ-glutamyl sélectionnés parmi γ-Glu-Val-Gly, γ-Glu-Abu-Gly et γ-Glu-Abu ;
(B) un ou plusieurs types d'acides dicarboxyliques choisis parmi l'acide L-tartrique et l'acide adipique.

2. Composition selon la revendication 1, dans laquelle le rapport de la quantité contenue de l'ingrédient (B) à la quantité contenue de l'ingrédient (A) est de 0,05 à 5000 en termes de rapport pondéral.

3. Composition selon la revendication 1 ou 2, dans laquelle la quantité contenue de l'ingrédient (A) est de 40 ppm (p/p) ou plus.

4. Composition selon l'une quelconque des revendications 1 à 3, dans laquelle l'ingrédient (A) est constitué de γ-Glu-Val-Gly.

5. Composition selon l'une quelconque des revendications 1 à 4, qui est un agent conférant le kokumi.

6. Composition selon l'une quelconque des revendications 1 à 5, qui est un agent conférant un goût initial de type kokumi.

7. Procédé de production d'un aliment ou d'une boisson, le procédé comprenant :
l'ajout des ingrédients suivants (A) et (B) à un aliment ou une boisson, ou à une matière première de celui-ci :
(A) un ou plusieurs types de peptides γ-glutamyl sélectionnés parmi γ-Glu-Val-Gly, γ-Glu-Abu-Gly et γ-Glu-Abu ;
(B) un ou plusieurs types d'acides dicarboxyliques choisis parmi l'acide L-tartrique et l'acide adipique.

8. Procédé selon la revendication 7, dans lequel le rapport de la quantité ajoutée de l'ingrédient (B) à la quantité ajoutée de l'ingrédient (A) est de 0,05 à 5000 en termes de rapport pondéral.

9. Procédé selon la revendication 7 ou 8, dans lequel l'ingrédient (A) est constitué γ-Glu-Val-Gly.

10. Procédé selon l'une quelconque des revendications 7 à 9, dans lequel l'ingrédient (A) est ajouté de sorte que la concentration de l'ingrédient (A) au moment de la consommation ou l'absorption soit de 0,0005 à 200 ppm (p/p).

11. Procédé selon l'une quelconque des revendications 7 à 10, dans lequel l'ingrédient (B) est ajouté de sorte que la concentration de l'ingrédient (B) au moment de la consommation ou de l'absorption soit de 0,1 à 5000 ppm (p/p).

12. Procédé selon l'une quelconque des revendications 7 à 11, dans lequel l'aliment ou la boisson à produire est un aliment ou une boisson imprégné de kokumi.

13. Procédé selon l'une quelconque des revendications 7 à 12, dans lequel l'aliment ou la boisson à produire est un aliment ou une boisson auquel on confère un goût initial de type kokumi.

14. Procédé pour conférer un goût initial de type kokumi à un aliment ou une boisson, le procédé comprenant :
l'ajout des ingrédients suivants (A) et (B) à l'aliment ou à la boisson, ou à une matière première de celui-ci :
(A) un ou plusieurs types de peptides γ-glutamyl sélectionnés parmi γ-Glu-Val-Gly, γ-Glu-Abu-Gly et γ-Glu-Abu;
(B) un ou plusieurs types d'acides dicarboxyliques choisis parmi l'acide L-tartrique et l'acide adipique.
